# EUROPÄISCHE PATENTSCHRIFT

(11) **EP 0 228 985 B1**
(45) Veröffentlichungstag und Bekanntmachung des Hinweises auf die Patenterteilung: **20.01.1993**
(21) Anmeldenummer: 86730145.9
(22) Anmeldetag: 17.09.1986
(51) Int. Cl.: A61N 1/365

(54) **Herzschrittmacher**
Heart pacemaker
Stimulateur cardiaque

(30) Priorität: 18.09.1985 DE 3533597
(43) Veröffentlichungstag der Anmeldung: 15.07.1987
(73) Patentinhaber: BIOTRONIK Mess- und Therapiegeräte GmbH & Co Ingenieurbüro Berlin, D-12359 Berlin (DE)
(72) Erfinder: Schaldach, Max, D-8520 Erlangen (DE)
(74) Vertreter: Christiansen, Henning, Dipl.-Ing.

(56) Entgegenhaltungen:
- EP-A- 0 012 195
- EP-A- 0 064 940
- EP-A- 0 140 472
- DE-A- 3 043 999
- DE-A- 3 240 430
- US-A- 4 515 161
- US-A- 4 523 593

## Beschreibung

Die Erfindung betrifft einen Herzschrittmacher mit Mitteln zur Beeinflussung der Stimulationsrate in Abhängigkeit von einem für das Schlagvolumen repräsentativen im Patientenkörper aufgenommenen Meßwert.

Ein derartiger Schrittmacher ist aus der EP-A-0 140 472 bekannt. Bei einem derartigen Schrittmachern besteht die Schwierigkeit, die Abhängigkeit der Rate von einer mit dem Schlagvolumen korrespondierenden Größe Patienten optimal einzustellen, da die kardialen Gegebenenheiten patientenindividuell verschieden sind.

Zur exakten Angleichung des Schrittmacherverhaltens an das Individuum ist die Kenntnis des nichtlinearen Zusammenhangs zwischen Herzzeitvolumen, Schlagvolumen, Herzrate und Belastung notwendig, wobei eine Steuerkennlinie oder ein Regelbereich ausgewählt werden muß. Das Verhalten des Herzens und insbesondere die Eignung des Schlagvolumens im Bereich der Belastungsgrenze als Steuerkriterium zu dienen, läßt sich nicht vorhersagen. Es sind daher zahlreiche Versuche nötig, um eine geeignete Einstellung zu erzielen.

Da eine derartige punktweise Überprüfung und Einstellung des Arbeitsbereiches relativ aufwendig ist, liegt der Erfindung die Aufgabe zugrunde, eine Anordnung anzugeben, welche es ermöglicht, die funktionale Abhängigkeit der Herzfrequenz vom Schlagvolumen auf einfache Weise zu optimieren und gegebenenfalls periodisch zu überprüfen.

Diese Aufgabe ist durch die im kennzeichnenden Teil des Anspruchs 1 angegebenen Maßnahmen gelöst.

Die Erfindung beruht insbesondere auf der Erkenntnis, daß die Eichung im Bereich höherer körperlicher Belastung ohne Schwierigkeiten möglich ist, da das Schlagvolumen sich wegen der Grenze der Adaptionsfähigkeit des Herzens nicht mehr ändert und somit die Belastung selbst nicht genau bekannt zu sein braucht und auch nicht meßtechnisch erfaßt werden muß.

Besonders vorteilhaft ist, daß im Normalfall auf eine punktweise Erfassung des sich einstellenden Herzzeitvolumens bei der Einstellung eines derartigen Schrittmachers im Bereich großer Belastung verzichtet werden kann, was eine wesentliche Erleichterung für den Patienten mit sich bringt. Auch ist kein Ergometer oder ähnliche Meßeinrichtung, wie sie bei der Aufnahme eines Belastungs-EKG notwendig sind, erforderlich. Die Anordnung kann in günstiger Weise sogar derart ausgebildet werden, daß regelmäßig selbsttätig eine Anpassung an wechselnde Randbedingungen bezüglich Herzverhalten und Lebensumständen erfolgt. Insbesondere folgt das System einer Besserung der Leistungsfähigkeit. Trotzdem ist eine Kontrolle und Neueinstellung auch durch den behandelnden Arzt möglich, der durch die Tendenz inzwischen eingetretener selbsttätiger Veränderungen eine diagnostische Hilfe erhält.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 Eine Perspektivzeichnung zur Funktion eines Schlagvolumen gesteuerten Schrittmachers,
Figur 2 ein bevorzugtes Ausführungsbeispiel nach der Erfindung in Blockdarstellung sowie
Figur 3 ein Diagramm zur Funktion des Ausführungsbeispiels.

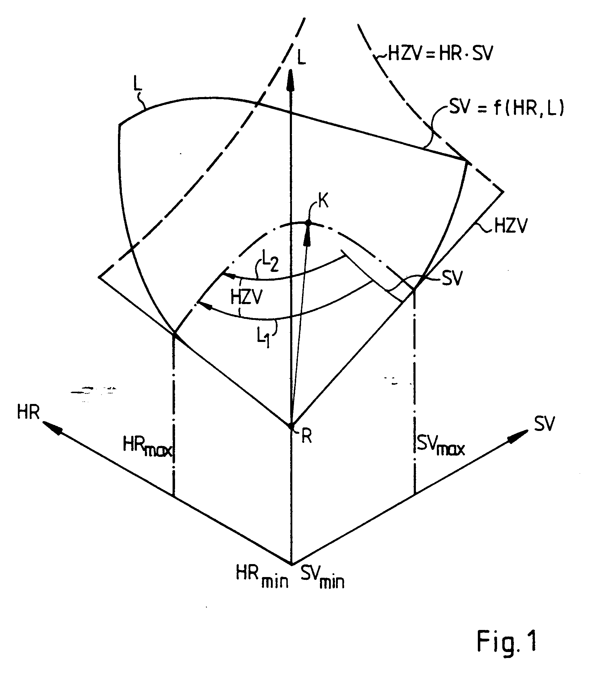

In Figur 1 ist in einem grafischen Darstellung das Herzzeitvolumen (HZV) in Abhängigkeit von der Herzrate und im Schlagvolumen angegeben. Diese Darstellung ist für die Steuerung des Schrittmachers von besonderer Bedeutung, da das Herzzeitvolumen ein Maß für die Leistungsfähigkeit des Herzens ist und die Veränderung der Herzrate kein ausreichendes Kriterium darstellt, wenn das erzielbare Schlagvolumen außer Betracht gelassen wird. Das HZV-Diagramm zeigt den rein mathematischen Zusammenhang, der sich rechnerisch durch Multiplikation ergibt. Das Schlagvolumen ist zwischen dem minimalen und maximalen Wert veränderbar, wobei entsprechendes für die Herzrate gilt. Beim Schrittmacher läßt sich jedoch nur die Herzrate beeinflussen, während das Schlagvolumen gemäß der vorhandenen Adaptionsfähigkeit des Herzens selbsttätig einstellt. Es liefert also einerseits als Produkt mit der Herzrate ein Maß für die aktuelle Leistung des Herzens, während andererseits eine Verkleinerung oder Vergrößerung bezogen auf eine konstante Herzrate ein Kriterium dahingehend bildet, daß das körperinterne Regelsystem eine Vergrößerung oder Verkleinerung des Herzzeitvolumens wünscht, soweit diese Adaptionsfähigkeit bei dem betreffenden Patienten noch vorhanden ist.

In einem weiteren DIagramm ist die Abhängigkeit des Schlagvolumens (SV) von der Herzrate (HR) und der Belastung (L) wiedergegeben, wobei die axiale Richtung entsprechend dem anderen Diagramm gewählt wurde, um einen vereinfachten Vergleich zu ermöglichen. Dabei zeigt sich, daß das Schlagvolumen bei beschränkter Adaptionsfähigkeit des Herzens zu höheren Werten hin begrenzt ist, wobei zu höheren Frequenzwerten sich das Schlagvolumen verkleinert, weil die Kammerfüllung sich verringert. Durch Vergleich der beiden Diagramme läßt sich nunmehr der Arbeitsbereich des Schrittmachers festlegen, wenn nämlich davon ausgegangen wird, daß die Last nicht wesentlich vom Herzzeitvolumen abweichen darf, wenn das Leistungsvermögen des Herzens der Belastung längerfristig adäquat sein soll.

Es zeigt sich also, daß eine zu starke Heraufsetzung der Herzfrequenz (Kennlinie mit geringer Steigung in bezug auf Achse HR) zu einem Absinken des Herzzeitvolumens führt und daher im dargestellten Fall nicht günstig ist. Günstig ist dagegen der eingezeichnete Verlauf einer Kennlinie, wobei die Belastung des betreffenden Patienten wegen der eingeschränkten Volumenadaption des Herzens begrenzt bleiben muß. Verläuft das Kennfeld dagegen zu höheren Frequenzen HR hin - wie gestrichelt dargestellt - so kann die Arbeitskennlinie flacher gewählt werden zu höheren Herzfrequenzen hin ist ein Bereich ohne Verringerung des Schlagvolumens erreichbar, bei dem das Herzzeitvolumen entsprechend vergrößert ist. Diesen Erkenntnissen trägt die erfindungsgemäße Anordnung Rechnung.

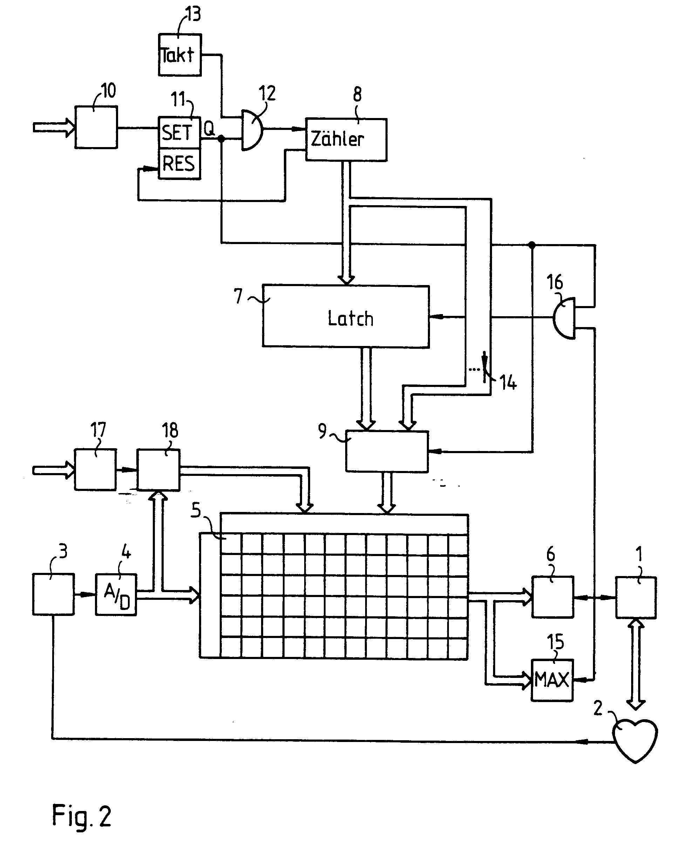

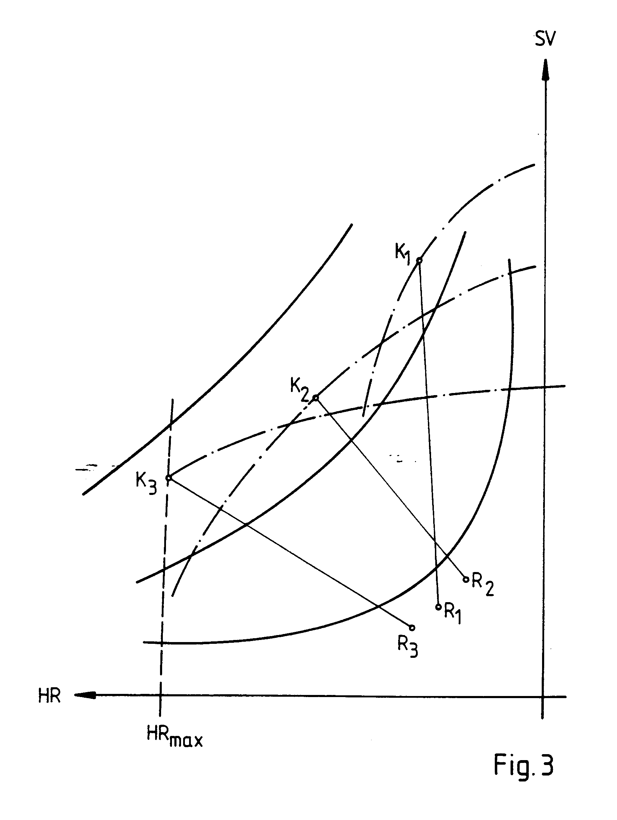

Die Erläuterung des erfindungsgemäßen Ausführungsbeispiels soll nunmehr anhand der Blockdarstellung gemäß Figur 2 vorgenommen werden, wobei auf das Diagramm nach Figur 3 bedarfsweise verwiesen wird.

In Figur 2 ist ein üblicher Schrittmacher vorgesehen, welcher mit einem schematisch dargestellten Herzen 2 in Wechselwirkung tritt. Der Schrittmacher weist die bekannten Maßnahmen auf, welche eine Konkurrenz der Stimulationsimpulse mit Herzeigenaktionen verhindern. Zusätzlich ist ein Sensor für eine mit dem Schlagvolumen korrelierte Größe vorgesehen, wie sie beispielsweise mittels Elektropletys mografie gewonnen werden kann. Ein entsprechender Meßwertaufnehmer 3 steuert einen Analog-Digital-Wandler 4 an, dessen Ausgangssignal einen Speicher 5 adressiert, welcher matrixartig organisiert ist, wobei im Falle einer üblichen Speicherstruktur - beipielsweise im Zusammenhang mit einem Mikroprozessorsystem - das Ausgangssignal des Analog-Digital-Wandlers 4 eines einen größeren Speicherbereich adressierenden Datenwortes bildet. Im normalen Betrieb werden durch die wechselnden digitalisierten Kammervolumenwerte zugehörige Frequenzwerte ausgelesen, welche einen digital ansteuerbaren Oszillator in seiner Frequenz beeinflussen. Der Oszillator 6 gibt die Grundrate des Schrittmachers vor und ist im Falle eines Demand-Schrittmachers durch Herzeigenaktionen rücksetzbar, so daß er das Escape-Intervall des Schrittmachers definiert.

Die gespeicherten Werte bestimmen eine Arbeitskennlinie, welche bevorzugt eine Gerade bildet und im Diagramm gemäß Figur 3, in dem der Zusammenhang zwischen Herzrate (HR) und Schlagvolumen (SV) dargestellt ist, eine der Geraden K₁-R₁ bis K₃-R₃ bilden. In den Diagrammen gemäß Figur 3 sind verschiedene Werte für das Herzzeitvolumen (HR x SV) als Hyperbeläste dargestellt, wobei das Herzzeitvolumen vom Koordinatenursprung aus zunimmt. Die Verbindungen R-k können auch eine gewisse Krümmung aufweisen, wenn ein derartiger Verlauf aus therapeutischen Gründen günstig erscheint.

Die Umschaltung der verschiedenen Kurven erfolgt bei dem in Figur 2 dargestellten Ausführungsbeispiel durch eine weitere Teiladresse, die in einem Latch 7 enthalten ist. Dieses Latch wird von einem Zähler 8 angesteuert, der normalerweise in Ruhe ist. Das Ausgangssignal des Latch 7 als Adressensignal gelangt zu einem Unschalter 9, der in seiner aktivierten Stellung auch das Ausgangssignal des Zählers 8 direkt zur Adressierung des Speicherbereiches 5 durchhalten kann. Im normalen Betriebszustand erfolgt die Adressierung des Speicherbereichs - wie gesagt - jedoch durch das Latch 7.

Die Eichung, d.h. die Neueinstellung der Arbeitskennlinie erfolgt bei dem Ausführungsbeispiel der Erfindung dann, wenn die körperliche Belastung des Patienten so groß ist, daß keine weitere Schlagvolumenadaption in Richtung auf Volumenzunahme mehr erfolgen kann (Steiler Bereich der Fläche L in Figur 1).

Da zu höheren Herzfrequenzen hin das Schlagvolumen zusätzlich durch die sich verringernde Kammerfüllung abnimmt, folgt die Abhängigkeit Schlagvolumen-Herzrate nicht mehr einem der Hyperbeläste - was einer Konstanthaltung des Herzzeitvolumens in Anpassung an die augenblickliche körperliche Belastung bedeuten würde - sondern folgt einer Kurve, welche mehr oder weniger einem der entgegengesetzt zu den Hyperbel gekrümmten Kurven durch die Punkte K₁ bis K₃ entspricht.

Um dem Patienten bei großer Belastung ein möglichst großes Herzzeitvolumen zur Verfügung zu stellen, wird mit der erfindungsgemäßen Anordnung für den Fall der großen körperlichen Belastung die Arbeitskennlinie so gelegt, daß ihr Endpunkt für den Fall großer Belastung (L) zu einem möglichst großen Herzzeitvolumen gehört, so daß durch die Variation der Herzfrequenz in Anpassung an das Schlagvolumen eine möglichst große Leistungsfähigkeit des Herzens gesichert ist. Die Variation der Herzrate wird beschränkt durch die obere zulässige Frequenz.

Erreicht der Patient also seine obere zumutbare Belastung, so wird ein erster Belastungssensor 10 aktiv und gibt ein Ausgangssignal ab. Dieser Belastungssensor besteht bevorzugt aus einem Sensor für die Bluttemperatur oder die Respirationsrate und ist digital auf einen bestimmten Schwellwert eingestellt, der gegebenenfalls durch äußere Schaltmittel veränderbar ist. Bei einem programmierbaren Schrittmacher ist das Ausgangssignal des Belastungssensors in entsprechender Weise auch aktiv durch Programmiermittel eingebbar, so daß die Eichung durch den Arzt kontrolliert vorgenommen werden kann. Bei einem vorhandenen Belastungssensor für einen oberen großen Belastungswert erfolgt die Anpassung des Schrittmachers an das Herzverhalten jedoch selbsttätig. Der Grenzwert des Belastungsgebers hingegen ist bevorzugt wieder durch äußere Programmiermittel patientenindividuell anpaßbar.

Die Aktivierung des Sensors 10 löst die Abgabe eines Impulses aus, wodurch ein Flip-Flop 11 gesetzt wird, so daß der Eichzustand ausgelöst wird. Mit dem Ausgangssignal Q des Flip-Flops 11 wird ein UND-Gatter 12 durchgeschaltet, welches das Taktsignal eines Generators 13 zu dein Zähler 8 durchläßt, welcher nunmehr einen Zählzyklus durchläuft. Durch das Signal Q wird auch der Umschalter 9 betätigt, so daß das Ausgangssignal 8 des Zählers nunmehr den Speicherbereich 5 direkt adressiert. Da das höchstwertige Bit der durch den Zähler gebildeten Teiladresse durch einen Inverter 14 invertiert wird, erfolgt ein Zugriff auf einen anderen Speicherbereich des Speichers 5, wobei hier jedoch die weitere Teiladresse durch den Analog-Digital-Wandler 4 abgegeben wird, so daß nunmehr in Abhängigkeit von der Position des Zählers 8 und des Analog-Digital-Wandlers 4 Speicherplätze adressiert werden. Diese Speicherplätze enthalten jeweils einen Frequenzwert, so daß mit dem Durchlauf des Zählers ein Frequenzbereich adressiert und langsam durchlaufen wird, der der hohen Belastung des Patienten entspricht. Bei der aus Anschauungsgründen gewählten matrixartigen Organisation des Speichers werden also mit dem Durchlauf des Zählers verschiedene Spalten der Matrix adressiert, wobei alle Speicherplätze einer Spalte den selben Wert zur Ansteuerung des Oszillators 6 enthalten (bei einem anderen bevorzugten Ausführungsbeispiel kann der den Oszillator ansteuernde Digitalwert durch eine entsprechende Umformung während des Eichvorgangs auch von der Ausgangsadresse des Zählers 8 hergeleitet werden, wobei der Adresseneingang des Zählers 8 während des Eichvorgangs entsprechend umgeschaltet werden muß).

Des weiteren enthält jeder adressierte Speicherplatz einen dem momentanen Herzzeitvolumen (Produkt aus HR und SV) entsprechenden Wert, wobei dieser Wert einem Maximumdetektor 15 zugeleitet wird. Dieser Maximumdetektor gibt jeweils einen Ausgangsimpuls ab, wenn ein neuer Wert größer war als der vorhergehende. Das Ausgangssignal ist im Falle des Eichbetriebs durch ein aktiviertes Unterteil 16 zum Latch 7 durchgelassen, so daß - wenn bei der Variation der Herzfrequenz ein größerer Wert für das derzeitige Zeitvolumene gefunden wird - der zugehörige Zählerstand in das Latch 7 eingespeichert wird. Nach dem vollständigen Durchlauf des Testzyklus gibt der Zähler 8 beim Erreichen seines Ausgangszustands einen Impuls ab, welcher das Flip-Flop 11 in seinen Ausgangszustand zurücksetzt. Damit ist der Suchlauf abgeschlossen und in das Latch 7 der Teiladresse der Kennlinie eingespeichert, welche zu demjenigen Punkt gehört, der im Falle einer großen Belastung des Patienten eine maximale Herzleistung sichert. Die Arbeitskennlinie wird dabei bevorzugt so gewählt, daß der ermittelte Arbeitspunkt für den Fall großer Belastung den Endpunkt der Arbeitskennlinie bildet.

Das dargestellte Ausführungsbeispiel stellt lediglich eine Möglichkeit der Realisierung dar, wobei der entsprechende Arbeitskennlinienverlauf auch durch entsprechende mathematische Operationen ermittelt werden kann. Das gilt auch für die Arbeitskennlinie. Die tabellenartige Ermittlung wurde bei dem Ausführungsbeispiel zum Zwecke der einfachen Darstellung gewählt und bietet Vorteile, wenn die Arbeitskennlinien keine Geraden sondern einen beliebig gekrümmten Verlauf haben sollen.

Der Zähler (8) für die testweise Variation der Herzfrequenz löst im Testzyklus selbstverständlich nur Herzfrequenzwerte aus, welche eine (programmierbare) obere zulässige Frequenzgrenze für den Patienten nicht überschreiten. Tritt bei dieser Frequenz bei großer Belastung noch kein Abfall des Schlagvolumens auf, so bildet die maximale Frequenz denjenigen Wert, der bei maximalem Schlagvolumen und großer Belastung eingestellt wird.

Dasjenige Ende der Arbeitsgeraden, welches dem Ruhezustand entspricht, ist ebenfalls programmierbar. Dabei kann durch einen zweiten Sensor für die Belastung 17 - gegebenenfalls automatisch - ein Ausgangssignal abgegeben werden, wenn der Patient in Ruhe ist, was durch Beschleunigungsaufnehmer etc. vom Schrittmacher erkannt werden kann. Das Ausgangssignal des Sensors 17 ist aber auch vom Arzt durch äußere Programmiermittel zu erzeugen.

Vom Detektor 17 wird - nachdem sich das zu der gewählten Stimulationsfrequenz gehörige Schlagvolumen eingestellt hat - ein Adressengeber 18 aktiviert, welcher durch Veränderung einer weiteren Teiladresse des den Speicher 5 adressierenden Gesamtadressenwortes festlegt, das im Ruhezustand eine vorgegebene Stimuationsrate eingehalten wird, wobei durch die Adressierung des betreffenden Speicherbereichs von allen Arbeitskennlinien, welche durch den zuvor ermittelten Punkt B führen, diejenige gewählt wird, die auch durch den nunmehr aufgefundenen Punkt R im Falle der Ruhe führt. (Diese Operation läßt sich ebenfalls durch ein entsprechendes Programm softwaremäßig durchführen.)

Auf diese Weise wird das sich im Ruhezustand einstellende Schlagvolumen der vorgegebenen Ruherate zugeordnet. Die schlagvolumenabhängige Frequenzadaption ist somit auch für den Ruhebereich "selbsteichend", so daß Meßwertschwankungen des Schlagvolumensensors ausgeglichen werden. Im Ruhebereich wird allerdings eine feste Stimulationsrate vorgegeben.

Die Erfindung beschränkt sich in ihrer Ausführung nicht auf das vorstehend angegebene Beispiel. Vielmehr sind eine Vielzahl von Varianten denkbar, welche von der dargestellten Lösung auch bei grundsätzlich anders gearteten Ausführungen Gebrauch machen. So ist kann es ebenfalls günstig sein eine Folge von Meßwertpaaren von Stimulationsrate und schlagvolumen nach den genannten Kriterium der Maximimierung des Herzeitvolumens zu ermitteln und eine Arbeitskennlinie zu interpolieren. Auf diese Weise wird sich bei der schlagvolumengesteuerten Ratenadaption stets ein Wertepaar Schlagvolumen/Stimulationsrate einstellen, das den Belastungsanforderungen am ehesten entspricht.

Insbesondere beschränkt sich die Ausführung nicht auf die Realisierung mit diskreten logischen Baugruppen, sondern läßt sich vorteilhaft auch mit programmierter Logik - insbesondere unter Verwendung eines Mikroprozessors - realisieren.

## Patentansprüche

1. Herzschrittmacher mit Mitteln zur Beeinflussung der Stimulationsrate in Abhängigkeit von einem für das Schlagvolumen repräsentativen im Patientenkörper aufgenommenen Meßwert,
**gekennzeichnet durch**
Schaltungsmittel mit einem ersten Betriebszustand und einem zweiten Betriebszustand,
Mittel zum Durchlaufen eines Stimulationsratenintervalls unter körperlicher Belastung des Patienten in dem ersten Betriebszustand, wobei das Stimulationsratenintervall der körperlichen Belastung angepaßt ist,
Multiplikationsmittel, welche jeweils einen vom Körper des Patienten abgeleiteten, für das Schlagvolumen repräsentativen Meßwert mit dem aktuellen Wert der Stimulationsrate multiplizieren,
Erkennungsmittel für den Maximalwert des Produkts und Speichermittel zum Festhalten mindestens eines Wertepaares von zugehöriger Stimulationsrate und für das Schlagvolumen repräsentativen Meßwert, sowie
Stimulationsmittel, welche in dem zweiten Betriebszustand beim Erscheinen des festgehaltenen für das Schlagvolumen repräsentativen Meßwert, Stimulationsimpulse mit der in den Speichermitteln festgehaltenen zugehörigen Rate auslösen.

2. Herzschrittmacher nach Anspruch 1, **dadurch gekennzeichnet,** daß ein oder mehrere Wertepaare Punkte einer Arbeitskennlinie sind, welche jeweils einem für das Schlagvolumen repräsentativen Meßwert einen Wert der Stimulationsrate mathematisch oder tabellarisch zuordnet.

3. Herzschrittmacher nach einem der vorangehenden Ansprüche, **dadurch gekennzeichnet,** daß für einen der körperlichen Ruhe entsprechenden Belastungszustand eine entsprechend niedrige Stimulationsrate dem sich dabei einstellenden mit dem Schlagvolumen korrelierten Meßwert vorgeben ist.

4. Herzschrittmacher nach Anspruch 3, **dadurch gekennzeichnet,** daß eine Arbeitskennlinie durch Interpolatiion zwischen dem der Belastung und dem der körperlichen Ruhe zugeordneten Wertepaare festgelegt wird.

5. Herzschrittmacher nach einem der vorangehenden Ansprüche, **dadurch gekennzeichnet,** daß ein Belastungssensor vorgesehen ist, der bei Erreichen eines vorgegebenen Werts ein den zweiten Betriebszustand auslösendes Signal abgibt.

6. Herzschrittmacher nach einem der vorangehenden Ansprüche, **dadurch gekennzeichnet,** daß der zweite Betriebszustand durch externe Programmiermittel auslösbar ist.

## Claims

1. A heart pacemaker with means for affecting the stimulation rate in dependence on a measured value received in the patient's body and representative of the beat volume,
**characterised by**
circuit means with a first operating condition and a second operating condition,
means for passing through a stimulation rate interval with the bodily loading of the patient in the first operating condition, in which the stimulation rate interval is matched to the bodily loading,
multiplication means which in each case multiply a measured value derived from the patient's body and representative of the beat volume by the current value of the stimulation rate,
means for recognising the maximum value of the product and storage means for retaining at least one pair of values of related stimulation rate and measured value representative of the beat volume, and
stimulation means which, in the second operating condition when the retained measurement value representative of the beat volume appears, stimulation pulses at the related rate retained in the storage means.

2. A heart pacemaker according to claim 1, **characterised in that** one or more pairs of values are points on an operating characteristic which associates in each case a value of the stimulation rate mathematically or in table form with a measured value which is representative of the beat volume.

3. A heart pacemaker according to any one of the preceding claims, **characterised in that** for a loading condition corresponding to the body at rest a correspondingly low stimulation rate is preset for the thereby arising measured value which is correlated to the beat volume.

4. A heart pacemaker according to claim 3, **characterised in that** an operating characteristic is determined by interpolation between the pairs of values associated with loading and the body at rest.

5. A heart pacemaker according to any one of the preceding claims, characterised in that a loading sensor is provided which when a predetermined value is reached emits a signal which triggers the second operating condition.

6. A heart pacemaker according to any one of the preceding claims, **characterised in that** the second operating condition can be triggered by external programming means.

## Revendications

1. Stimulateur cardiaque comportant des moyens pour influencer la fréquence de stimulation en fonction d'une valeur de mesure détectée sur le corps du patient et représentative du débit systolique,
stimulateur caractérisé
par des moyens de commutation présentant un premier mode de fonctionnement et un second mode de fonctionnement,
par des moyens pour parcourir une plage de fréquences de stimulation en tenant compte de la charge corporelle du patient dans le premier mode de fonctionnement, étant précisé que la plage de fréquences de stimulation est adaptée à la charge corporelle,
par des moyens de multiplication qui multiplient chaque fois une valeur de mesure, détectée sur le corps du patient et représentative du débit systolique, par la valeur actuelle de la fréquence de stimulation,
par des moyens d'identification de la valeur maximale du produit et des moyens de mémorisation pour mémoriser au moins une paire de valeurs constituée de la fréquence de stimulation correspondante et de la valeur de mesure représentative du débit systolique, ainsi que
par des moyens de stimulation qui, dans le second mode de fonctionnement, lors de l'apparition de la valeur de mesure mémorisée, représentative du débit systolique, provoquent des impulsions de stimulation présentant la fréquence correspondante, mémorisée dans les moyens de mémorisation.

2. Stimuiateur cardiaque selon la revendication 1, caractérisé par le fait qu'une ou plusieurs paires de valeur constituent des points d'une caractéristique de travail qui, à chaque valeur de mesure représentative du débit systolique, fait correspondre, par calcul mathématique ou par un tableau, une valeur de la fréquence de stimulation.

3. Stimulateur cardiaque selon l'une des revendications précédentes, caractérisé par le fait qu'à un état de charge correspondant au repos corporel est affectée une faible fréquence de stimulation correspondant à la valeur de mesure alors obtenue et en corrélation avec le débit systolique.

4. Stimulateur cardiaque selon la revendication 3, caractérisé par le fait que l'on établit une caractéristique de travail par interpolation entre la paire de valeurs correspondant à la charge et la paire de valeurs correspondant au repos corporel.

5. Stimulateur cardiaque selon l'une des revendications précédentes, caractérisé par le fait qu'il est prévu un détecteur de charge qui, lorsque l'on atteint une valeur prescrite, émet un signal provoquant le second mode de fonctionnement.

6. Stimulateur cardiaque selon l'une des revendications précédentes, caractérisé par le fait que le second mode de fonctionnement peut être provoqué par un moyen de programmation extérieur.
